Europäisches Patentamt

European Patent Office

Office européen des brevets

(11) Publication number: **0 266 907**
A2

## (12) EUROPEAN PATENT APPLICATION

(21) Application number: 87308839.7

(22) Date of filing: 06.10.87

(51) Int. Cl.4: **A61N 1/42** , A61N 1/32 ,
A61N 1/04 , A61N 1/40 ,
A61N 1/36

(30) Priority: 09.10.86 GB 8624227

(43) Date of publication of application:
11.05.88 Bulletin 88/19

(84) Designated Contracting States:
CH DE FR GB IT LI SE

(71) Applicant: THERAFIELD HOLDINGS LIMITED
St. Georges Chambers 1 Athol Street
Douglas, Isle of Man(GB)

(72) Inventor: Fellus, Victor Marcel
19 Rue Du Dr.Arnaudet
F-82190 Meudon(FR)

(74) Representative: Gray, Robin Oliver et al
BARON & WARREN 18 South End Kensington
London W8 5BU(GB)

(54) Electrotherapeutic apparatus.

(57) Apparatus is provided for emitting high frequency electromagnetic waves (51) to a patient's body and comprises an antenna (5) operatively coupled to a switched pulsed high frequency source (3) and to a source (6) of electric lesser frequency pulsed signals (50) at a frequency within a range such that electromagnetic radiation from the antenna (5,10) has a transcutaneous nerve stimulation effect. The source (6) of pulsed electric signals being adapted to operate at a lesser pulse rate than that of the source (3) of high frequency signals (51). Clock means (8) is provided for controlling switching means (4,7) whereby the antenna (5) is arranged to radiate a series of electric pulses (50) at the lesser frequency, and between said electric pulses (50) to radiate trains of pulses (51) of the electromagnetic radiation at the high frequency.

The apparatus provided enhancement of the pain relief effect of the transcutaneous nerve stimulation so that it persists for a substantially longer period.

Fig.1

Fig.8

Xerox Copy Centre

## 'ELECTROTHERAPEUTIC APPARATUS'

It is known to provide electrotherapeutic apparatus for emitting high frequency electromagnetic waves, in particular to a patient's body, and including an antenna for applying the electromagnetic radiation. For example, there is disclosed in US Patent specification 4 197 851 apparatus comprising low voltage power supply means, circuit means for producing high frequency electrical signals, said circuit means having an input and an output, means for connecting said input to said power supply means, and at least one antenna which is applicable directly to a patient's body and which is connected or connectible to the output of said circuit means, for producing at the antenna a low energy high frequency electromagnetic field without significant thermal effects on the body, said antenna comprising at least one conductor which is carried by a support of an electrically insulating material and which includes a length of conductive material arranged in a predetermined pattern. Suitably the antenna output is not more than 10 mW per square cm and the circuit means includes means for producing low frequency pulses having an input connected to said power supply means and an output, and a pulse shaping stage having an input connected to the output of the means for producing low frequency pulses and an output to a high frequency stage including a high frequency emitter controlled to a preselected frequency. The apparatus is suitably set up to produce low frequency pulses of high frequency electromagnetic radiation from the antenna at a repetition frequency of between 1 and 10,000Hz, with a pulse duration of between 10 to 100 microseconds and an UHF frequency of radiation of for example 27.125± 0.125 MHz.

Such apparatus has been highly successful in therapeutic application and in particular the alleviation or cure of a condition requiring treatment by successive periods of treatment of a patient over an extended period.

Since the apparatus causes no sensation to the patient there can be psychological uncertainty in the patient's mind that he is receiving treatment or that the apparatus is functioning, and it is a main object to provide means to provide such sensation as will overcome such uncertainty.

It is known since the eighteenth century to provide electronic apparatus for transcutaneous electrical nerve stimulation as a method of relieving pain in a patient. Such apparatus works by delivering low intensity electrical signals through the skin by means of electrodes applied to the skin to stimulate the nervous system of a patient. Such apparatus is adapted to operate within a higher low-frequency range of 10-120Hz or a lower low-frequency range of 2-9 Hz and it is thought that within the higher frequency range the signals activate the nervous system to block pain signals from the brain, and in the lower range to cause the release of the body's own analgesics which are natural morphine-like substances called endomorphines or enkeptalins. The electric signals are applied in pulses spaced apart by between a few milliseconds and a half second. The current intensity, pulse rate and the pulse length are suitably adjustable so that the stimulation mode best suited to a patient may be set.

Such apparatus has proved successful in use for example in relieving pain in post-surgical recovery but it is not known to have any health beneficial or long-lasting effect and in use has proved to require repeated treatment of a patient in continuous pain at relatively short intervals over an extended period under medical supervision.

It is an object to obtain the advantages of both systems whereby a patient's condition requiring improvement is not only alleviated over an extended period of exposures at intervals and has a sensation of such treatment, but also that pain relief is provided at each interval of treatment.

Electrotherapeutic apparatus emitting high frequency electromagnetic energy according to the invention comprises antenna means for applying high frequency electromagnetic radiation operatively connected via switching means to a source of pulsed high frequency signals, said high frequency being within a range such that said radiation is able to improve certain conditions and to a source of electric lesser frequency pulsed signals, said lesser frequency being within a range such that electromagnetic radiation from said antenna has a transcutaneous nerve stimulation effect, said source of pulsed electric signals being adapted to operate at a lesser pulse rate than that at which the source of high frequency signals is adapted to operate and clock means being provided for controlling said switching means whereby said antenna means is arranged to radiate a series of electric pulses at said lesser frequency and between said electric pulses to radiate trains of pulses of electromagnetic radiation at said high frequency.

It has been found that by appropriate predetermination of the high frequency pulses and of low frequency electric pulses, not only is the known health improvement and the known transcutaneous nerve stimulation obtained but also that the pain relief effect of the nerve stimulation is substantially enhanced to the extent that it persists for a substantially longer period after an interval of treatment than hitherto, such that the frequency of treatment for pain relief can substantially be reduced with advantage to the patient and reduction in the medical supervision required over an extended period.

Suitably the switching means is settable such that either one of the signal generators may be operated without the other, and the signal generators are suitably separately adjustable so that signal intensity, pulse rate and pulse width may be varied in each case for adjusting the therapeutic treatment and nerve stimulation of a patient to the patient's requirements.

In an example the source of electric signals has been arranged to operate at a repetition frequency of 100Hz with a pulse width of a millisecond and at 80 volts driving the antenna through a 1-10 transformer and the high frequency signal source has been arranged to operate between 5 and 150MHz, using quartz, or transistor driven oscillators suitable for generating all possible harmonics, at a pulse width of between 10 and 200 microseconds and a repetition frequency of between 1 and 10,000 Hz to give a signal of between 0.3 and 10 volts per square cm at the antenna.

The antenna means is suitably formed with a central laminar conductive zone or electrode for coupling to the low frequency electric source and supplying the low frequency electrical energy, and a surrounding laminar zone antenna for coupling to the high frequency source, and is suitably a flexible or rigid printed circuit which may be double-sided with the central zone forming an electric conducting pad on one side and the high frequency surrounding antenna on the opposite side.

The invention will now be described, by way of example, with reference to the accompanying, partly diagrammatic drawings, in which:-

Figure 1 is a schematic block diagram of apparatus according to the invention,

Figure 2 is a plan view of an antenna for use with apparatus according to the invention;

Figure 3 is a circuit diagram of part of the apparatus adapted to generate electric low frequency pulsed signals for the transcutaneous nerve stimulation function.

Figure 4 is a representation of the form of the electrical pulses from the Figure 3 circuit;

Figure 5 is a block circuit diagram of part of the apparatus of Figure 1 adapted to generate high frequency pulsed signals;

Figure 6 is a representation of signal pulses from the circuit of Figure 5;

Figure 7 is an embodiment of the circuit of Figure 5, and

Figure 8 is a representation of the composite low and high frequency outputs from the antenna means.

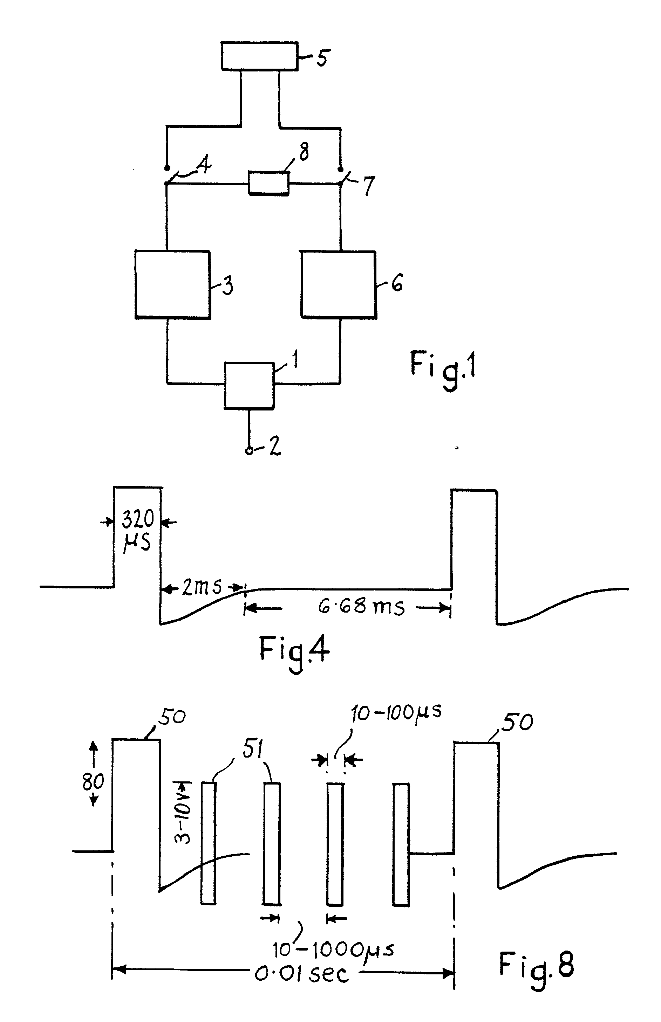

Referring first to Figure 1 the apparatus comprises a power supply 1 fed from a low voltage, suitably 9 volt cell 2 and supplying power to a high frequency generator 3 adapted to generate pulsed signals at a predetermined high frequency of 5 to 150 MHz with all harmonics, and at a pulse rate of between 1 and 10,000Hz, a pulse width of between 10 and 100 microsecond and pulse height between 0.3 and 10 volts per square cm at the antenna, the signals being output through switching means 4 to an antenna 5. The power supply 1 also supplies power to an electric low frequency generator 6 adapted to generate pulsed signals at a frequency of the order of 100Hz with a pulse width of 90 milliseconds at 80 volts which are fed to the antenna 5 via a 1-10 transformer and switching means 7. The switching means 4 and 7 are driven by a clock circuit 8 to open and close alternately i.e. in opposite phase to each other whereby the antenna means 5 is either driven by one or other of the generators 3 and 6.

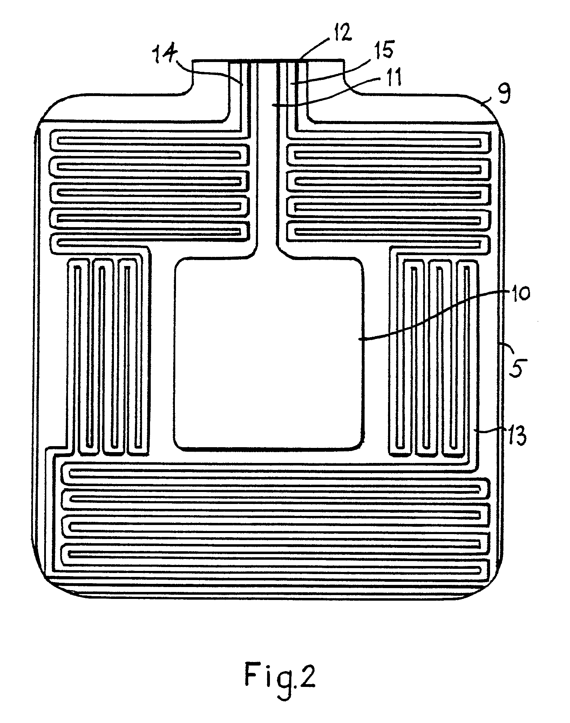

The antenna 5 is suitably a flexible or a rigid printed circuit and an example is shown in Figure 2 which comprises a flexible insulating laminar 9 formed with a central conductive laminar zone 10 defining an electrode for the electric low frequency signals from generator 6 with a conductive track 11 leading therefrom to a connector portion 12 of the printed circuit. Surrounding the central zone 10 the printed circuit is formed with a conductive track 13 extending in sinuous fashion about the central zone 10 from ends 14,15 at opposite side of the conductive track 11 at the connector portion 12 of the printed circuit. The sinuous form is such that the track 13 extends substantially entirely through the area of the laminar 9 outside the central zone for aereal distribution of radiated electromagnetic energy to a patient. Electromagnetic sinuous track 19 and electrically conductive area 10 are suitably realised on opposite sides of a double-sided printed circuit, the conductive zone 10 necessarily being in contact with the skin of a patient being treated and the high frequency electromagnetic track 19 inducing a capacitive effect through an intervening insulating layer.

The connector portion 12 is adapted for connection via a suitable edge connector to the switch means 4 and 7 to connect the antenna portions 10,13 respectively to the switch means 4,7.

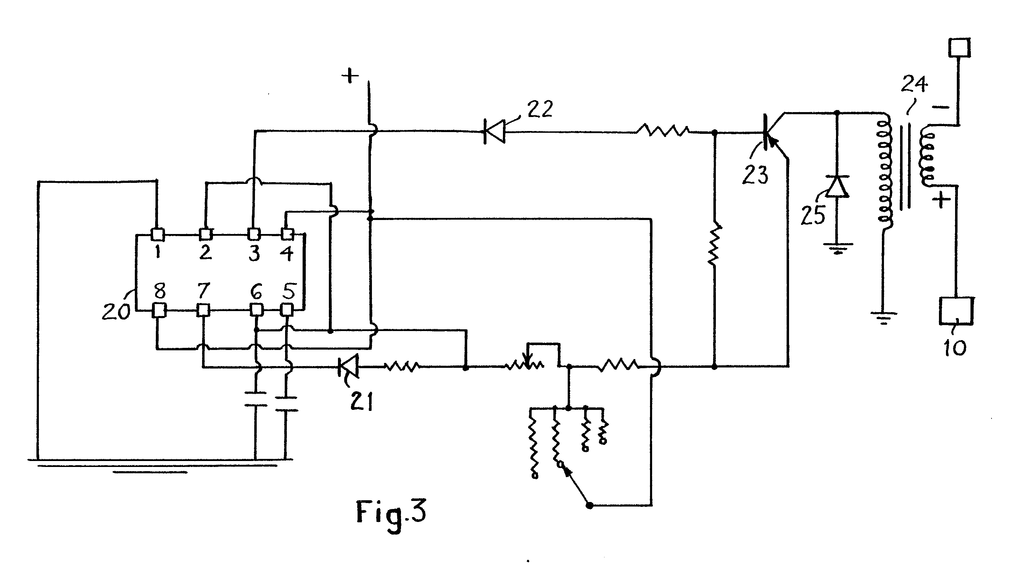

Referring now to Figure 3 the circuit for generating the electric low frequency signal, i.e. the generator 6 of Figure 1, comprises an oscillator oscillating at 100Hz by means of an integrated circuit device 20 such as an LM 555 or TB 555 and having contact pads 1-8. The generated wave from the oscillator is taken from pad 7 and is shaped through a diode 21 e.g. IN 4148 and 180 ω resistor, and from pad 3 through a diode 22, e.g. IN 4148 and a 220 ω resistor to an output PNP transistor 23 e.g. 2N5322 having its collector connected to the primary of an output transformer 24 of ratio 1-10 to give 220 volts under a few mA. The secondary of the transformer 24 is connected to appropriate contacts of the antenna on positive side and to a conductive electrode pad on the negative side, the electrode pad being for application to the skin of the patient in addition to the electrode 10 of the antenna. The collector of output transistor 23 is also earthed via a diode 25 type BAV21 with its cathode to the collector side in order to avoid the negative side of the burst wave following a pulse. The number 2 pad of the IC package 20 is connected to pad 6 and through a 2.2μF condenser to earth, and also for the square wave shaping to the 180 ω resistor following the diode 21. Pad 5 of the IC package is connected to earth via a 10 μF condenser acting as a voltage regulator. DC power is applied from a low voltage source e.g. from a 9 volt battery cell to pads 4 and 8 of the IC package and pad 1 is earthed.

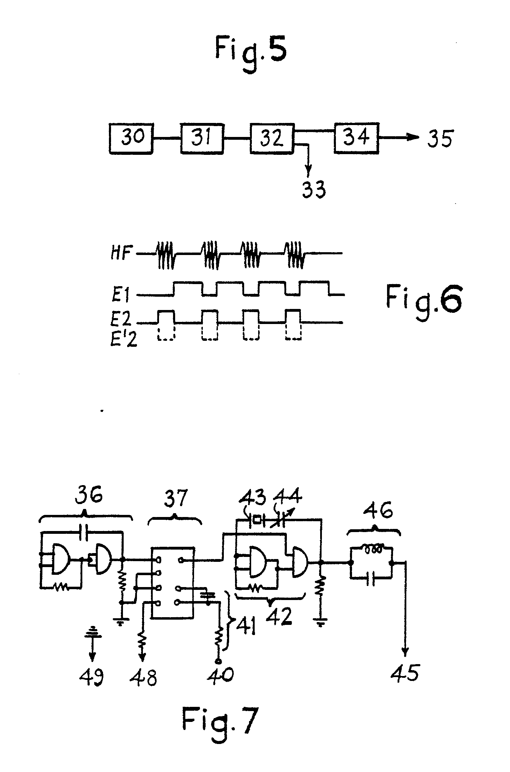

The pulse form generated at the collector of transistor 23 is typically as shown in Figure 4 comprising a positive squared wave pulse followed by a negative burst wave which is drained to earth through the diode 25, BAV21.

The circuit for producing the high-frequency electromagnetic waves will now be described with reference to Figure 5. The circuit is of solid state form and comprises a multivibrator 31, a pulse shaper 32 and a high frequency stage 34 which are suitably disposed in a housing. The multivibrator 31 has an input which is connected to the low-voltage power supply means 30. The multivibrator generates low-frequency electrical signals which are fed via its output to the input of the pulse shaper 32 which shapes these signals. The shaped electrical signals are connected at a terminal 33 forming one output of the pulse shaper and are fed through another output of the pulse shaper to the high-frequency stage 34 which produces high-frequency electromagnetic waves which are transmitted through its output terminal 15 to the antenna 5 at the high frequency track 13.

If the HF signals are transmitted in regular successive trains (first waveform in Figure 5), as they are in a preferred embodiment of the invention, the electrical signals emitted at 13 may be of the type shown at E1, i.e. trains of pulses.

As regards the high frequency, operations take place for example at 27.125 ± 0.125 MHz, which is within one of the bands set aside for high-frequency medical use.

The length of the HF wave trains may be of the order of 10 to 100 microseconds, and the repetition frequency of the successive trains may be of the order of 1 Hz to 10,000 Hz.

As regards the electrical signals, the voltage applied to the electrodes antenna 12 and 14 is generally of the order of 3 to 10 volts and preferably of the order of 5 to 6 volts, the intensity of the current flowing in the part to be treated being very low.

Figure 8 shows in more detail the circuit components of the multivibrator 31, pulse shaper 32 and HF stage 34 in the block diagram of Figure 4. The oscillator assembly 36 governs the frequency of the HF wave trains and/or of the successive electrical signals and is made up of conventional elements as shown by the conventional representations thereof. There are many integrated circuits which can be used as oscillators of this kind. The signals are transmitted to a device 37 to allow the HF and electrical signals to be shaped. The device 37 may be a 74,121 integrated circuit (international coding; such an integrated circuit is made inter alia by Sescosen and Texas Instruments). At terminals 10,11 of the 174,121 circuit is inserted an assembly 41 for regulating the length of the HF wave trains or the electrical signals 40 being connected to the power supply.

From terminal 6 of the 74,121 circuit are emitted signals which are passed to an HF emitter 42 which is quartz controlled by crystal 43 to the selected frequency set by capacitor 44. The HF wave trains are transmitted to the antenna at terminal 45 via an LC circuit 46 for matching to the antenna. If 36 corresponds to 31 (Figure 5) 37 and 41 correspond to 32, and 42,43,44 and 46 correspond to 34. Terminals 38 and 39 correspond to 33, and terminal 45 corresponds to 35 (Figure 5).

In Figure 6, electrical pulses whose sign is opposite from the pulses shown in solid lines are shown in broken lines at E'2. This illustrates the possibility of emitting in all cases, be they E1, E2 or others, either positive going pulses, or negative going pulses, or alternating pulses.

The HF antenna will always be insulated from the patient by means of, for example, a simple lacquer with which they are coated or an insulating material sheath or pouch, whereas when electrodes are used the best possible electrical contact needs to be provided with the patient. When for example antennae and electrodes are printed on the same support, they can be placed on either side of the support, which will allow the antenna when situated on the opposite side from the patient to be electrically insulated.

The composite output from the antenna will be of the form shown in Figure 8 where a series of pulses 50 of electric low frequency signals at 100Hz are interspaced by trains of pulses 51 of high frequency radiation.

## Claims

1. Electrotherapeutic apparatus emitting high frequency electromagnetic energy (51) comprising antenna means (5,13) for applying high frequency electromagnetic radiation (51) operatively connected via switching means (4) to a source (3) of pulsed high frequency signals, said high frequency being within a range such that said radiation is able to improve certain conditions, and via switching means (7) to a source (6) of electric lesser frequency pulsed signals (50), said lesser frequency being within a range such that electromagnetic radiation from said antenna has a transcutaneous nerve stimulation effect, said source (6) of pulsed electric signals being adapted to operate at a lesser pulse rate than that at which the source (3) of high frequency signals is adapted to operate and clock means (8) being provided for controlling said switching means (4,7) whereby said antenna means (5) is arranged to radiate a series of electric pulses (50) at said lesser frequency and between said electric pulses (50) to radiate trains of pulses (51) of electromagnetic radiation at said high frequency.

2. Apparatus as claimed in claim 1, characterised in that switching means (4,8) are settable such that either one of the signal generators (3,6) may be operated without the other, and the signal generators (3,6) are separately adjustable so that signal intensity, pulse rate and pulse width may be varied in each case for adjusting the therapeutic treatment and nerve stimulation of a patient to the patient's requirements.

3. Apparatus as claimed in claim 1, characterised in that the source (6) of electric signals (50) of lesser frequency is arranged to operate at a repetition frequency of 100Hz with a pulse width of a millisecond and at 80 volts driving the antenna (5,10) through a 1-10 transformer and the high frequency signal source (3) is arranged to operate between 5 and 150MHz, using quartz, or transistor driven oscillators suitable for generating all possible harmonics. at a pulse width of between 10 and 200 microseconds and a repetition frequency of between 1 and 10,000Hz to give a signal of between 0.3 and 10 volts per square cm at the antenna (5,13).

4. Apparatus as claimed in claim 1, characterised in that antenna means (5) is formed with a central laminar conductive electrode zone (10) for coupling to the low frequency electric source (6) and supplying the low frequency electrical energy (50), and a surrounding laminar zone antenna (13) for coupling to the high frequency source (3).

5. Apparatus as claimed in claim 4, characterised in that the antenna means (5) is a laminar printed circuit having the central zone (10) forming an electric conducting pad on one side and the high frequency surrounding antenna (13) on the opposite side.

6. Apparatus as claimed in claim 5, characterised in that printed circuit (5) is flexible.

7. Apparatus as claimed in claim 6, characterised in that the high frequency antenna (13) surrounding the central zone conducting pad (10) comprises a sinuous conductive track extending substantially entirely throughout the area of the laminar outside the central zone (10).

Fig.1

Fig.4

Fig.8

Fig.2

Fig.3

0 266 907

# Fig.5

30 — 31 — 32 — 34 → 35

33

HF

E1

E2

E'2

# Fig.6

36    37    43 44    46

41    42

49    48    40    45

# Fig.7